# EUROPEAN PATENT APPLICATION

(11) **EP 4 119 678 A1**
(43) Date of publication of application: **18.01.2023**
(21) Application number: 21768641.9
(22) Date of filing: 02.03.2021
(51) Int. Cl.: C12Q 1/6813, C12Q 1/6837, C12Q 1/6851, C12Q 1/686, G01N 33/53

(54) **METHOD FOR ASSESSING ONSET OF MYOCARDITIS OR RISK THEREOF**

(30) Priority: 13.03.2020 JP 2020044279
(71) Applicant: TERUMO Kabushiki Kaisha, Tokyo 151-0072 (JP)
(72) Inventor: HIRAHARA, Ichiro, Ashigarakami-gun, Kanagawa 259-0151 (JP); SAKATA, Kiyoshi, Ashigarakami-gun, Kanagawa 259-0151 (JP); SATO, Hideki, Ashigarakami-gun, Kanagawa 259-0151 (JP); TAKASAKI, Hidenori, Ashigarakami-gun, Kanagawa 259-0151 (JP)
(74) Representative: Casalonga
(86) International application number: PCT/JP2021/007823
(87) International publication number: WO 2021/182170

(57) **Abstract**

Provided is a means capable of determining the development of myocarditis or the risk thereof by a simple and minimally invasive method. A method of determining the development of myocarditis or risk thereof, wherein it is determined that myocarditis is developed or there is a risk thereof when a concentration or expression amount of ADAM28 in a body fluid sample collected from a subject is reduced compared with a reference value.

## Description

### TECHNICAL FIELD

The present invention relates to a method of determining a development of myocarditis or a risk thereof.

### BACKGROUND ART

Myocarditis is an inflammatory disease developing mainly in the myocardium, and it is called pericardial myocarditis when the inflammation reaches the pericardium (Guidelines for Diagnosis and Treatment of Acute and Chronic Myocarditis (2009 Revision)). Since the definitive diagnosis is difficult in mild cases, the details of the incidence and mortality rates of myocarditis in Japan are unclear. However, there are reports that the frequency of myocarditis is 115 per 100,000 people (Okada R, Kawai S, Kasuya H. Jpn Circ J 1989;53(1):40-48.) and that 0.6% of non-cardiac death autopsy cases are asymptomatic myocarditis (Feeley KM, Harris J, Suvarna SK. J Clin Pathol 2000;53:147-149.). Furthermore, 20% of sudden deaths at age of 40 or younger are said to be caused by myocarditis. Thus, myocarditis is by no means a rare disease.

There are different types of myocarditis. For example, myocarditis is classified into acute myocarditis and chronic myocarditis, depending on the mode of development and the disease course. Among acute myocarditis, those that fall into a cardiopulmonary crisis at the initial stage of the disease are especially called fulminant myocarditis (Aoyama N, Izumi T, Hiramori K, et al. Circ J 2002;66:133-144.). Meanwhile, chronic myocarditis has a prolonged type and a subclinical type. Many of the causes are infections, and the pathogens of the infections are known to be mostly viruses, and also bacteria, rickettsia, spirochete, mycoplasma, fungi, protozoa, parasites, and the like (Guidelines for Diagnosis and Treatment of Acute and Chronic Myocarditis (2009 Revision)). Other causes include physical stimuli, such as drugs, radiation, and heat; immune abnormalities, such as metabolic disorders and autoimmune diseases; and further pregnancy. However, it is not uncommon that the cause of the disease is unknown, and in such cases where the etiology is not identified, the myocarditis is diagnosed as idiopathic myocarditis.

In many patients with acute myocarditis, cold-like symptoms (chills, fever, headache, muscle pain, general malaise) and gastrointestinal symptoms, such as eating disorders, nausea, vomiting, diarrhea, or the like are preceded, then several hours to several days later, cardiac symptoms appear (Guidelines for Diagnosis and Treatment of Acute and Chronic Myocarditis (2009 Revision)). The cardiac symptoms may be associated with (1) signs of heart failure (about 70% frequency), (2) chest pain due to pericardial stimulation (about 44%), and (3) cardiac block or arrhythmia (about 25%). However, in myocarditis, few cases have obvious clinical symptoms and signs, and their initial symptoms and signs are nonspecific. It is not uncommon that a person having mere cold or digestive symptoms may develops into severe shock, syncope, and dyspnea in a very short period due to a decrease of cardiac function, and even develops into a fatal course of fulminant myocarditis. Fulminant myocarditis is said to have a lethality of about 50%. In addition, extensive myocardial inflammation leads to dilated cardiomyopathy-like symptoms with decreased cardiac functions, and heart failure may be repeated. In the chronic phase, the pumping power of the heart is reduced due to extensive pericardial fibrogenesis caused by inflammation, and a unique right heart failure called systolic pericarditis develops.

As described above, not only does myocarditis often have no preceding subjective symptoms, but it may also rapidly worsen, with development into a shock state presenting with blood pressure reduction, impaired consciousness and the like, and even cause sudden death. Thus, there are demands for a diagnostic method that can easily determine myocarditis in the early stage. In physical findings, it is important to check fever, pulse abnormalities (tachycardia, bradycardia, arrhythmia), hypotension, and the like, and examine gallop rhythm (appearance of a third heart sound), signs of pulmonary congestion such as moist rales, and signs of right heart failure such as jugular venous distention and lower leg edema. In cases of pericarditis complications, cardiac tamponade may appear. In addition to these symptoms and diagnostic findings, myocarditis is diagnosed by chest X-ray, electrocardiogram, echocardiogram, cardiac MRI, blood tests, and the like. In the blood biochemical examination, it is known that C-reactive protein (CRP) level is elevated, and blood concentration of myocardial proteins, such as aspartate aminotransferase (AST), lactate dehydrogenase (LDH), myocardial-specific creatinine kinase (CK-MB), and myocardial troponin are increased. However, CRP, AST, and LDH are also increased due to common inflammations other than myocarditis. CK-MB and myocardial troponin are blood markers that are specific and sensitive to myocardial injury because they are released into blood by myocardial necrosis and their blood concentrations are increased. However, their values become high when myocardial necrosis occurs in a wide area, and thus are high in myocardial infarction and acute coronary syndrome, leading to hardness in differential diagnosis of myocarditis. Furthermore, CK-MB is mainly present in cardiac muscles, but also slightly present in skeletal muscles, thus the value is also elevated in skeletal muscle diseases, such as rhabdomyolysis, multiple myositis, and muscular dystrophy. For this reason, cardiac catheterization, and even endocardial myocardial biopsy (myocardial biopsy) during cardiac catheterization, may be performed to confirm that the cause is not coronary artery disease, such as angina or myocardial infarction. Myocardial biopsy is an invasive and burdensome test for the patient. However, it is useful in determining treatment strategy and predicting the disease course because it may confirm the diagnosis and determine a type of myocarditis. Thus, myocardial biopsy may be repeatedly performed and followed up despite the invasiveness, when the disease is prolonged (Myocardial Biopsy: Class I, Level C, Guidelines for Diagnosis and Treatment of Acute and Chronic Myocarditis (2009 Revision)). In a case of decreased cardiac blood beat function (systolic function) or cardiac dilatation (diastolic function) failure, B-type natriuretic peptide (BNP) and N-terminal (NT)-proBNP in blood are measured as indicators of cardiac load. If heart failure is suspected from subjective symptoms and physiological findings, it is recommended to perform blood sampling tests including BNP and NT-proBNP values, urinalysis, electrocardiogram, chest radiograph, and the like, and investigate the validity of the diagnosis (Cardiomyopathy Practice Guidelines (2018 Revision)). In particular, as being established as an adjunct diagnostic method for heart failure, BNP is also useful in determining hypertrophic cardiomyopathy (Yoshibayashi M, Kamiya T, Saito Y, et al. N Engl J Med 1993;329:433-434.). BNP is reported to be associated with not only left ventricular hypertrophy and left ventricular pressure difference (Mizuno Y, Yoshimura M, Harada E, et al. Am J Cardiol 2000;86:1036-1040.)(Hasegawa K, Fujiwara H, Doyama K, et al. Circulation 1993;88:372-380.), but also severity of heart failure (Maron BJ, Tholakanahalli VN, Zenovich AG, et al. Circulation 2004;109:984-989.), exercise tolerance (Thaman R, Esteban MT, Barnes S, et al. Am J Cardiol 2006;98:515-519.), and even clinical prognosis (Cardiomyopathy Practice Guidelines (2018 Revision)). Practice guidelines for acute and chronic heart failure also recommend that subjective symptoms, history, family history, physical findings, electrocardiogram, and chest X-ray are first investigated in the diagnosis of heart failure, and then, when chronic heart failure is suspected, the blood BNP/NT-proBNP values should be measured (Acute and Chronic Heart Failure Practice Guidelines (2017 Revision)). However, blood BNP/NT-proBNP values are indicators for heart failure, and their validity in earlier pathologies, such as myocarditis, before cardiac functions decrease, is not certain.

Meanwhile, when symptoms due to heart failure (chest pain, shortness of breath, dyspnea) or symptoms due to arrhythmia (palpitation, dizziness) are observed, an electrocardiogram test is performed. Electrocardiogram is a sensitive and simple diagnostic method, and even when the initial electrocardiogram change is minor, abnormal findings may become clear over time. Thus, in patients with suspected myocarditis, it is important to repeat electrocardiogram test over time. ST-T abnormality occurs most frequently, but it sometimes exhibits localized ST elevation, which is very similar to the abnormality in acute myocardial infarction, thus it is sometimes difficult to differentiate from myocardial infarction. Recently, it has been reported that viewing the properties of myocardium by cardiac MRI is useful as an auxiliary diagnosis. However, tests using such a modality are labor intensive and expensive, thus not suitable as a primary test for the disease.

### SUMMARY OF INVENTION

Accordingly, an object of the present invention is to provide a means capable of determining a development of myocarditis or a risk thereof, in particular myocarditis with mild cardiomyopathy before cardiac function decreases, by a simple and minimally invasive method.

The present inventors have conducted intensive studies to solve the above-described problems. As a result, the relationship between the development of myocarditis and ADAM28 has been surprisingly revealed for the first time. Based on this finding, the present inventors have completed the present invention.

That is, according to one embodiment of the present invention, there is provided a method of determining a development of myocarditis or a risk thereof, wherein it is determined that myocarditis is developed or there is a risk thereof when a concentration or expression amount of ADAM28 in a body fluid sample collected from a subject is reduced compared with a reference value.

According to another embodiment of the present invention, there is provided a method of providing data for determining a development of myocarditis or a risk thereof, including determining that myocarditis is developed or there is a risk thereof when a concentration or expression amount of ADAM28 in a body fluid sample collected from a subject is reduced compared with a reference value.

### DESCRIPTION OF EMBODIMENTS

One embodiment of the present invention relates to a method of determining a development of myocarditis or a risk thereof, wherein it is determined that myocarditis is developed or there is a risk thereof when a concentration or expression amount of ADAM28 in a body fluid sample collected from a subject is reduced compared with a reference value.

Another embodiment of the present invention relates to a method of providing data for determining a development of myocarditis or a risk thereof, including determining that myocarditis is developed or there is a risk thereof when a concentration or expression amount of ADAM28 in a body fluid sample collected from a subject is reduced compared with a reference value.

According to the present invention, a development of myocarditis or a risk thereof, in particular myocarditis with mild cardiomyopathy before cardiac function decreases, can be determined by a simple and minimally invasive method.

Specifically, according to the method of one embodiment of the present invention, a development of myocarditis or a risk thereof in a subject can be easily determined. Furthermore, according to the method of one embodiment of the present invention, mild myocardial injury in which cardiac function has not decreased and BNP and NT-proBNP have not been elevated can be detected. Furthermore, since the method requires only measuring a concentration or expression amount of ADAM28 in a body fluid sample, the test for determination can be performed simply. When a blood sample is used as the body fluid sample, it can be diagnosed with a portion of the blood taken by a routine blood test, and thus there is little burden on the subject. Thus, the method according to one embodiment of the present invention can provide determining and data for determining the development of myocarditis or the risk thereof in a simple and minimally invasive manner without using expensive modalities, and can be expected to provide significant prognostic improvements for myocardial injury.

Hereinafter, embodiments of the present invention will be described, but the present invention is not limited to the following embodiments.

As used herein, a range indicated by "X to Y" means "X or more and Y or less". Unless otherwise specified, measurements of procedures, physical properties, and the like are performed under conditions of room temperature of 20 to 25°C and relative humidity of 40 to 50% RH.

As used herein, the "subject" is a subject, preferably a human subject, who may have progression of myocarditis or myocardial injury. In another preferred embodiment, the subject is a patient diagnosed as suffering from a disease involving myocarditis or a patient treated for the disease after having the diagnosis.

As used herein, the "body fluid sample" is any body fluid from a living body (organism) in which a measurement of the concentration or expression amount of ADAM28 can be performed. The body fluid sample is preferably a blood sample. The blood sample may be whole blood, plasma, or serum. As the blood sample, the blood (whole blood) collected from a subject may be used as is, or a sample supplemented with an additive, such as EDTA potassium salt, heparin, sodium citrate for the purpose of preventing coagulation or the like may be used. The timing for collecting a body fluid sample from a subject is not particularly limited. When the body fluid sample is a blood sample, the blood sample may be arterial blood or venous blood.

The body fluid sample used in the method according to the present invention is preferably used for measurement immediately after collected from a subject, but may be used for measurement after storage. For example, the storage method of the blood sample is not particularly limited as long as the value of the concentration or expression amount of ADAM28 in the sample is not changed. For example, in a long-term storage, it is preferred that the sample be cryopreserved at -80°C.

In the method according to the present invention, the measurement of the value of the concentration or expression amount of ADAM28 can be performed using conventional known methods as described below.

ADAM proteins (ADAMs: a disintegrin and metalloproteinases) are multifunctional proteins involved in ectodomain shedding of transmembrane proteins, and cell adhesion and infiltration (Edwards DR, Handsley MM, Pennington CJ. Mol Aspects Med 2008;29(5):258-289.)(Murphy G. Semin Cell Dev Biol 2009;20 (2):138-145.). The human genome contains 25 ADAMs including 4 pseudo-genes, and 21 ADAMs are composed of 13 proteolytic ADAMs having proteolytic activity and 8 non-proteolytic ADAMs (Edwards DR, Handsley MM, Pennington CJ. Mol Aspects Med 2008;29(5):258-289.)(Shiomi T, Lemaitre V, D'Armiento J, Okada Y. Pathol Int 2010;60(7):477-496.). Proteolytic ADAMs share the metalloproteinase domain of matrix metalloproteinases (MMPs), and typical proteolytic ADAMs include propeptide, metalloproteinase, disintegrin-like, cysteine-rich, epidermal growth factor-like, transmembrane and intracellular domains. Many proteolytic ADAMs, including ADAM8, ADAM9, ADAM12, ADAM15, ADAM17, ADAM19, and ADAM28, are overexpressed in human cancers and known to be associated with cancer proliferation and progression. However, it has not been suggested to date that ADAM28 can be a useful indicator for assessment of myocardial inflammation and injury, particularly myocarditis in the early stage.

ADAM28 is a known protein, and the amino acid sequence and cDNA sequence thereof are also known. There are two types of ADAM28: a membrane-type (ADAM28m) and a secretory type (ADAM28s). Representative amino acid sequence and cDNA sequences of human membrane-type ADAM28 (and mature forms thereof) and representative amino acid sequence and cDNA sequences of human secretory-type ADAM28 (and mature forms thereof) are disclosed in WO2016/143702. Note that the "human membrane-type ADAM28" means that the amino acid sequence or nucleotide sequence of membrane-type ADAM28 has an amino acid sequence or nucleotide sequence identical or substantially identical to the amino acid sequence or nucleotide sequence of membrane-type ADAM28 naturally expressed in humans. The "substantially identical" means that the amino acid sequence or nucleotide sequence of interest has 70% or more (preferably 80% or more, more preferably 90% or more, further preferably 95% or more, most preferably 99% or more) identity to the amino acid sequence or nucleotide sequence of membrane-type ADAM28 naturally expressed in humans and has the function of human membrane-type ADAM28. The same applies to non-human biological species and proteins other than membrane-type ADAM28, genes, and fragments thereof.

As used herein, the "expression amount" of ADAM28 is intended to include both the expression of an RNA (transcription product) that is complementary to a gene encoding ADAM28 and the expression of ADAM28 (translation product) itself. Thus, as used herein, the expression amount of ADAM28 refers to the expression amount or expression intensity of the transcription product or translation product. The expression amount can usually be analyzed by the production amount of the transcription product, or the production amount, activity or the like of the translation product. The "concentration" of ADAM28 also means the abundance (expression amount) of ADAM28 per unit volume of a body fluid sample (e.g., a blood sample).

The expression amount of ADAM28 may be measured by measuring a transcription product of a gene encoding ADAM28, that is, mRNA or by measuring a translation product from the gene, that is, ADAM28 itself. Preferably, the measurement is performed by measuring a translation product of the gene. Note that the transcription product of the gene includes cDNA obtained by reverse transcription from mRNA.

Measurement of expression amount of the translation product can be performed by quantifying ADAM28 or measuring activity of ADAM28. Examples of the quantification method of ADAM28 include an electrophoresis method, a Western blotting method, a chromatography method using affinity chromatography, ion-exchange chromatography, gel filtration chromatography, reverse phase chromatography, immunochromatography, or the like, and a method for measuring mass spectrum. The measurement can be performed simply and accurately by using an antibody specific to ADAM28.

The antibody can be produced by known methods. Alternatively, the antibody is available from, for example, MyBioSource, Inc., Invitrogen, Santa Cruz Biotechnology, Inc., Sigma-Aldrich Co. LLC, or the like, and can be obtained and used as appropriate. The antibody for detection may be a polyclonal antibody or monoclonal antibody.

Detection of ADAM28 using an antibody can be performed by, but is not limited to, an immunochromatography method, a Western blotting method, an EIA method, an ELISA method, an RIA method, a flow cytometry method, or the like. The antibody can be labeled by a fluorescence label, a radioactive label, an enzyme, biotin, or the like, and a secondary antibody for detection labeled as such can be used. Currently, in clinical practice, the presence of myocardial injury is assessed by detecting myocardial troponin T in blood samples by immunochromatography method (Trop T Sensitive, Roche Diagnostics K.K.). Thus, similarly in the method according to the present invention, by measuring ADAM28 by immunochromatography method, it is expected that a myocarditis evaluation method that is clinically minimally invasive and allows simple measuring, that can be universalized at a low cost, that is highly accurate and reproducible, and that is capable of standardizing the measurements or the like, is established. Further, as a result, it is expected that appropriate treatment can be administered, and heart failure and sudden death can be prevented. Note that the above measurement by immunochromatography method is preferably performed with an immunochromatography test strip. It is preferred that the immunochromatography test strip has a sample pad region that receives a blood sample, a conjugate pad region containing a labeled antibody capable of binding to ADAM28, a membrane body on which a specimen is deployed, and a test line in which an antibody capable of binding to ADAM28 is fixed on the membrane body and the antibody is allowed to bind to ADAM28 bound by the labeled antibody in the conjugate pad region. Furthermore, it is more preferred that the immunochromatography test strip has a control line that can determine the amount of labels bound in the test line described above. Note that it is further preferred that the labels are those that develop stronger color depending on the concentration.

The expression amount of the transcription product may be measured, for example, by using a nucleotide containing whole or a portion of a nucleotide sequence of mRNA as a probe or primer to measure the extent of gene expression in a sample. For example, the measurement can be performed by a method using a microarray (microchip), a Northern blot method, a quantitative PCR method, or the like. As the quantitative PCR method, an agarose gel electrophoresis method, a polyacrylamide gel electrophoresis method, a fluorescence probe method, an RT-PCR method, a real-time PCR method, an ATAC-PCR method, a Taqman PCR method, a SYVER(registered trademark) green method, a Body Map method, a serial analysis of gene expression (SAGE) method, and a micro-analysis of gene expression (MAGE) method are known, and these methods can be appropriately used. Next-generation sequencers may also be used for evaluation. These methods can be used to measure the amount of mRNA with a nucleotide probe or primer that hybridizes to the mRNA. The nucleotide length of the probe or primer used for the measurement is preferably 10 to 50 mers, more preferably 15 to 25 mers.

When simultaneously measuring expressions of multiple genes, particularly expressions of genes of several types, it is preferable to use a DNA microarray. A DNA microarray can be produced by fixing nucleotides consisting of a portion of a nucleotide sequence of the gene or containing a portion thereof on an appropriate substrate. Examples of the substrate for fixing include a glass plate, a quartz plate, and a silicon wafer. Examples of the size of the substrate include 3.5 mm x 5.5 mm, 18 mm x 18 mm, and 22 mm x 75 mm, but it can be variously set depending on the number of spots of the probe and the size of the spots on the substrate. Examples of the method for fixing a polynucleotide or fragment thereof include a method of electrostatically coupling the nucleotide using the charge onto a solid phase support which is surface-treated with a polycation such as polylysine, polyethyleneimine, or polyalkylamine, and, a method of covalently bonding a nucleotide in which a functional group such as an amino group, an aldehyde group, a thiol group, or biotin is introduced to a solid phase surface in which the functional group such as an amino group, an aldehyde group, or an epoxy group is introduced. Immobilization may be performed using an array machine. The type and amount of mRNA can be determined by immobilizing at least one gene or a fragment thereof to a substrate to prepare a DNA microarray, contacting a subject-derived mRNA or cDNA labeled with a fluorophore with the DNA microarray to make hybridization, and measuring the fluorescence intensity on the DNA microarray.

In the method according to the present invention, the concentration or expression amount of ADAM28 measured by the method described above may be compared with a reference value to determine a development of myocarditis or a risk thereof. That is, a method of determining a development of myocarditis or a risk thereof is disclosed herein. Also disclosed herein is a method of providing data for determining a development of myocarditis or a risk thereof. In the methods, it is determined that myocarditis is developed or there is a risk thereof when the concentration or expression amount of ADAM28 is reduced compared with a reference value, as described above.

As used herein, the "reference value" is a numerical value that may serve as a reference for determining an elevation or reduction in the concentration or expression amount of ADAM28 in the method according to the present invention. Cancer is currently known as a disease in which blood ADAM28 level is elevated. Thus, a measurement value of the concentration or expression amount of ADAM28 in a person (e.g., a healthy subject) who has not been diagnosed with cancer can be taken as a reference value. For example, Kuroda et al. reported that a cut-off value of 1.72 ng/mL could differentially diagnose non-small cell lung cancer with a sensitivity of 72.5% and a specificity of 76.5% (Kuroda H, Mochizuki S, Shimoda M, et al. Int J Cancer 2010; 127(8): 1844-1856.). Since the measurement results may vary depending on the measurement procedure and the standard product used for the measurement, it is important to set the cut-off value in each measurement system. The concentration or expression amount of ADAM28 measured before development of cancer in the past in the same subject may also be used as a reference value. It is preferred that the reference value be the concentration or expression amount of ADAM28 in a body fluid sample (e.g., a blood sample) taken from a healthy subject.

According to further another embodiment of the present invention, a detection reagent for use in the method according to one embodiment of the present invention described above is provided. Also, a kit for use in the method according to one embodiment of the present invention described above, including the detection reagent, is provided. The detection reagent is one that enables a detection of the concentration or expression amount of ADAM28 in the body fluid sample described above. Examples of such a detection reagent include an antibody for specific binding to ADAM28 in a body fluid sample or a DNA or RNA for hybridization to an mRNA of an ADAM28 gene in a body fluid sample. Such a DNA or RNA may be a probe capable of detecting hybridization using a fluorescent label or the like. Alternatively, the DNA or RNA may be a primer that can be used to amplify an mRNA. The kit can include, in addition to the detection reagent described above, other reagents required for detection, such as a buffering agent, various nucleotides, other reagents required for hybridization or antibody binding, and the like.

### EXAMPLES

The effects of the present invention will be described using the following Examples and Comparative Examples, but the technical scope of the present invention is not limited to the following Examples.

### [Preparation of myosin administration solution]

A 10 mg/mL myosin solution was prepared by adding 0.01 M phosphate buffer to myosin (myosin, from calcium-activated porcine heart, Sigma-Aldrich Co. LLC). 20 mg of Mycobacterium Tuberculosis H37 Ra, desiccated (Nippon Becton Dickinson Company, Ltd.) while being ground using a mortar and pestle, was stirred into 2 mL of Mycobacterium Tuberculosis H37 Ra, Complete Adjuvant in Oil (Nippon Becton Dickinson Company, Ltd.) (hereinafter, referred to as CFA). The myosin solution and CFA were each taken into a 2 mL luer-lock all-plastic disposable syringe by 1 mL each, and the syringes were connected with a stopcock adapter for syringe. The two liquids were mixed in syringes until becoming cloudy to prepare a myosin administration solution.

### [Creation of Animal Model]

Myocarditis is often caused by infections of bacteria, viruses, or the like, but it is also known to be caused by autoimmunity (Guidelines for Diagnosis and Treatment of Acute and Chronic Myocarditis (2009 Revision)). Meanwhile, it has been reported that viral myocarditis evolves into autoimmune myocarditis and exacerbates myocardial injury (Richer MJ, et al. J Innate Immun 2009;1(5):421-434.) (Eriksson U, et al. Nat Med 2003;9(12):1484-1490.).

Myocarditis is believed to progress as follows (Martinez NE, et al. Future Virol 2012;7(6):593-608.). First, viral infection and proliferation in the heart cause congenital immune responses, and myocardial cells are damaged (Stage I: viral myocarditis). When the clearance of the virus is incomplete, myocardial injury occurs due to viral proliferation and anti-viral immunity, and autoantibodies and autoimmune T-cell reactions caused by epitope spreading and molecular mimicry occur (Stage II: Immune myocarditis). Low-grade virus survival and myocardial tissue injury in the first and second stages lead to cause cardiac remodeling and consequently dilated cardiomyopathy (Stage III: dilated cardiomyopathy).

Since even myocarditis caused by infection in this way evolves into autoimmune myocarditis and exacerbates myocardial injury, autoimmune myocarditis has been studied using animal models. As autoantigens that cause myocarditis, myosin, actin, ADP/ATP carrier proteins, SRA proteins, laminins, and the like are known, and myocardial myosin which is most abundant as proteins constituting myocardium is commonly used for preparation of a model by administering myocardial myosin to Lewis rats to elicit immunity (Izumi Tohru et al. Shinzo 1993; 25(7): 821-832). In this model, epicarditis and cardiac enlargement start about 1 week after myosin administration, and histologically, inflammatory cell infiltration into the myocardium and necrosis of the myocardium begin to appear. At week 3, myocarditis develops in all sensitized cases, and in some of them, the spread of myocarditis transmurally can be observed. Myocarditis becomes most severe on day 28, then cell infiltration fades out and is largely replaced with fibrogenesis after 42 days (Izumi Tohru et al. Shinzo 1993; 25(7): 821-832). In addition, in myosin myocarditis models, it has been reported that cardiac function (left ventricular fractional shortening, left ventricular ejection fraction) decreases at week 4 to 5 after myosin administration (Nagaya N, et al. Circulation 2005;112(8):1128-1135.)(Mina Y, et al. J Cardiovasc Pharmacol Ther 2013;18(2):152-161.).

In the experiment, a six-day quarantine and conditioning period was set for rats (Lewis, males, 7 weeks old, purchased from Charles River Laboratories Japan, Inc.), and after confirming that there were no abnormalities in the health status of all rats and no weight loss, the rats were subjected to the test. The rats were allowed to freely access food and water in a rearing environment with 12-hour lighting, a temperature of 20 to 26°C, and a humidity of 30 to 70%.

In the myosin administration group, 0.1 mL of the myosin administration solution (hereinafter also referred to as "antigen") was administered to the footpad of one hind limb of the rat under 2% isoflurane inhalation anesthesia. One week after the first administration, a second antigen administration was performed. At 2 and 5 days after the first antigen administration, an inactivated pertussis bacteria suspension (200 billion bacteria per mL, NACALAI TESQUE, INC.) was intraperitoneally administered at a dose of 0.1 mL/rat under awake condition. Before the administration, abdominal hairs were removed. The control group was a group of untreated rats, and the mean value of the control group was used as a reference value.

The experiments were conducted in accordance with the guidelines for animal experiments by Terumo Corporation.

### [Cardiac function test]

Three weeks after the first antigen administration, the left ventricular short-axis image (papillary muscle level) was depicted in M mode under 2% isoflurane inhalation anesthesia with an ultrasonic diagnostic device, and LVDd (left ventricular end-diastolic diameter) and LVDs (left ventricular end-systolic diameter) were measured. Then, the left ventricular ejection fraction (EF) was calculated by Teichholtz method, and the left ventricular fractional shortening (FS) was calculated by the formula of (LVDd - LVDs)/LVDd. As a result, no significant difference was observed in both FS and EF, confirming that cardiac function had not decreased yet (Tables 1 and 2). Statistical analysis was performed by Student's t-test, and p < 0.05 was considered significant.

### [Table 1]

**[Table 1] Left ventricular fractional shortening (FS) at autopsy in control group and myosin administration group**

| | Left ventricular fractional shortening (FS) (%) | | |
|---|---|---|---|
| | Mean value | Standard error | p |
| Control group | 59.11 | 0.69 | 0.56 |
| Myosin administration group | 57.42 | 1.51 | |

### [Table 2]

**[Table 2] Left ventricular ejection fraction (EF) at autopsy in control group and myosin administration group**

| | Left ventricular ejection fraction (EF) (%) | | |
|---|---|---|---|
| | Mean value | Standard error | p |
| Control group | 92.16 | 0.48 | 0.50 |
| Myosin administration group | 90.93 | 0.93 | |

### [Rat autopsy]

The day after the cardiac function test, the rats were weighed and then subjected to abdominal opening under 2% isoflurane inhalation anesthesia. Blood was collected from the abdominal aorta and vena cava into blood collection tubes containing EDTA-2K, and the rats were euthanized. The body weight at this time was significantly low in the myosin administration group (Table 3).

### [Table 3]

**[Table 3] Body weight at autopsy in control group and myosin administration group**

| | Body weight(g) | | |
|---|---|---|---|
| | Mean value | Standard error | p |
| Control group | 291.44 | 3.29 | 0.02 |
| Myosin administration group | 239.80 | 9.19 | |

Blood was centrifuged at 3000 rpm x 10 min at 4°C to collect plasma. Plasma was stored at -30°C and then subjected to NT-proBNP and ADAM28 measurements. The heart was rinsed with saline to remove blood, then weighed, and immersed in and fixed with 10% neutral buffered formalin. No significant difference was observed in the heart weight at this time between the two groups. After fixation, paraffin-embedded was performed, then myocardial degeneration/necrosis and mononuclear cell infiltration were evaluated by HE staining, and fibrogenesis was evaluated by Masson trichrome staining. The evaluation was scored by grades of four stages, and semi-quantified, then statistically analyzed. Statistical analysis was performed by Student's t-test, and p < 0.05 was considered significant.

Grades and scores were set as shown in Table 4 below.

### [Table 4]

**[Table 4] Grades and scores of myocardial pathological findings**

| Findings | Grade | Score |
|---|---|---|
| No findings | - | 0 |
| Very mild | + | 1 |
| Mild | + | 2 |
| Moderate | ++ | 3 |
| Severe | +++ | 4 |

As a result, myocardial degeneration/necrosis, mononuclear cell infiltration, and fibrogenesis were significantly observed in the myosin administration group, confirming that myocarditis had developed. The results of pathological findings are shown in Table 5.

### [Table 5]

**[Table 5] Average score value of myocardial pathological findings in control group and myosin administration group**

| | Pathological findings | Control group | | Myosin administration group | | t-test |
|---|---|---|---|---|---|---|
| | | Average score | Standard error | Average score | Standard error | p |
| Left ventricle | Myocardial degeneration/ necrosis | 0 | 0 | 1.3 | 0.33 | < 0.005 |
| | Mononuclear cell infiltration | 0 | 0 | 1.7 | 0.33 | < 0.001 |
| | Fibrogenesis | 0 | 0 | 1.3 | 0.33 | < 0.005 |
| Right ventricle | Myocardial degeneration/ necrosis | 0 | 0 | 2.2 | 0.31 | < 0.001 |
| | Mononuclear cell infiltration | 0 | 0 | 2.8 | 0.47 | < 0.001 |
| | Fibrogenesis | 0 | 0 | 1.5 | 0.22 | < 0.001 |

### [Measurement of NT-proBNP concentration]

The NT-proBNP concentration in plasma collected at autopsy was measured using Rat N-Terminal Pro-Brain Natriuretic Peptide ELISA (MyBioSource, Inc.) according to the manual. The results are shown in Table 6. Statistical analysis was performed by Student's t-test, and p < 0.05 was considered significant.

As a result, no significant difference was observed in the plasma NT-proBNP concentration between the myosin administration group and the control group.

### [Table 6]

**[Table 6] Plasma NT-proBNP concentration of arterial blood in control group and myosin administration group**

| | NT-proBNP concentration (ng/L) | | |
|---|---|---|---|
| | Mean value | Standard error | p |
| Control group (n=5) | 479 | 11 | 0.26 |
| Myosin administration group (n=3) | 458 | 8 | |

### [Determination of myocarditis]

In the myosin administration group, inflammation was observed in the pathological findings, but cardiac function did not decrease, and the plasma NT-proBNP concentration was not significantly different from that in the control group. Thus, it was determined that the inflammation was an early stage of myocarditis before reaching to heart failure.

### [Measurement of ADAM28 Concentration]

Concentrations of ADAM28 in plasma collected at autopsy were measured using Rat A Disintegrin and Metalloprotease 28 (ADAM28) ELISA Kit (MyBioSource, Inc.) according to the manual. The results are shown in Table 7. Statistical analysis was performed by Student's t-test, and p < 0.05 was considered significant.

As shown in Table 7, the myosin administration group had a significantly lower plasma ADAM28 concentration than the control group. Thus, it can be determined that myocarditis is developed or there is a risk thereof when the concentration (or expression amount) of ADAM28 is reduced compared with that in a healthy subject.

Note that Pearson correlation coefficient test for the concentrations of ADAM28 in arterial blood and venous blood collected from the same individual resulted in the correlation coefficient R of 0.87 and p < 0.001. From the results, it was determined that both arterial blood and venous blood can be used as a specimen.

### [Table 7]

**[Table 7] Plasma ADAM28 concentration of arterial blood in control group and myosin administration group**

| | ADAM28 concentration (ng/mL) | | |
|---|---|---|---|
| | Mean value | Standard error | p |
| Control group (n=5) | 1.84 | 0.07 | <0.05 |
| Myosin administration group (n=3) | 1.31 | 0.03 | |

This application is based on Japanese Patent Application No. 2020-044279 filed on March 13, 2020, the disclosure of which is incorporated herein by reference in its entirety.

## Claims

1. A method of determining a development of myocarditis or a risk thereof, wherein it is determined that myocarditis is developed or there is a risk thereof when a concentration or expression amount of ADAM28 in a body fluid sample collected from a subject is reduced compared with a reference value.

2. A method of providing data for determining a development of myocarditis or a risk thereof, comprising determining that myocarditis is developed or there is a risk thereof when a concentration or expression amount of ADAM28 in a body fluid sample collected from a subject is reduced compared with a reference value.

3. The method according to claim 1 or 2, wherein the reference value is a concentration or expression amount of ADAM28 in a body fluid sample collected from a healthy subject.

4. The method according to any one of claims 1 to 3, wherein the body fluid sample is a blood sample.

5. A detection reagent for use in the method according to any one of claims 1 to 4, which is capable of detecting a concentration or expression amount of ADAM28 in the body fluid sample.

6. The detection reagent according to claim 5, being an antibody against the ADAM28 or a DNA or RNA for hybridization to an mRNA of ADAM28 gene.

7. A kit for use in the method according to any one of claims 1 to 4, comprising the detection reagent according to claim 6.
